# EUROPEAN PATENT APPLICATION

(11) **EP 3 861 865 A1**
(43) Date of publication of application: **11.08.2021**
(21) Application number: 19869509.0
(22) Date of filing: 04.10.2019
(51) Int. Cl.: A23L 17/00, A23L 33/10

(54) **FUNCTIONAL FOOD COMPOSITION USING PROCESSED ANCHOVY PRODUCT AND MANUFACTURING METHOD THEREFOR**

(30) Priority: 05.10.2018 KR 20180118893; 27.03.2019 KR 20190034752
(71) Applicant: Jang, Young Jin, Gwangju 62011 (KR)
(72) Inventor: LEE, Ho Woo, Gwangju 62012 (KR); JANG, Young Jin, Gwangju 62011 (KR)
(74) Representative: Hauck Patentanwaltspartnerschaft mbB
(86) International application number: PCT/KR2019/013060
(87) International publication number: WO 2020/071867

(57) **Abstract**

The present invention relates to a functional food composition using an anchovy processed product and a method of manufacturing the same. More specifically, the present invention comprises the steps of: preparing a raw material food composition including semi-dried anchovies and raw anchovies; salting the raw material food composition; fermenting the salted food composition under a certain condition; extracting effective ingredients from the fermented food composition; purifying the extracted effective ingredients; and freeze-drying the purified effective ingredients.

## Description

### [Technical Field]

The present invention relates to a functional food composition using an anchovy processed product and a method of manufacturing the same. More specifically, the present invention relates to a functional food composition comprising an anchovy processed product as an effective ingredient, and has functional properties including improved blood circulation, improved blood triglycerides, improved blood cholesterol, increased tryptophan, and antioxidative activity, and a method of manufacturing the same.

### [Background Art]

In modern society, metabolic diseases are increasing due to a convenient living environment due to rapid automation, excessive nutritional intake due to an increase in processed foods and eating out, and a decrease in physical activity.

Meanwhile, blood circulation means that blood moves to each part of the body through blood vessels, and blood supplies oxygen and nutrients to each tissue of the body, and removes waste products created by cells.

In addition, it carries hormones necessary for our body, protects cells from external harmful substances, maintains proper body temperature, and maintains homeostasis in the body.

Therefore, good blood circulation is very important for maintaining body functions, and it is known that the incidence of cerebrovascular diseases such as arteriosclerosis and stroke is increased when blood circulation is impaired.

The main factors affecting blood circulation include chronic diseases such as high blood pressure and diabetes, eating habit, lifestyle, and genetic factors. The higher the standard of living and the westernization of the eating habit, the higher the incidence of cerebrovascular diseases related to blood circulation disorders, thereby causing socioeconomic problems.

Hyperlipidemia refers to a condition in which lipids such as free cholesterol, cholesterol esters, phospholipids, and triglycerides abnormally increase in the blood.

Hyperlipidemia usually does not show symptoms by itself, but if there is a lot of fat in the blood, it can stick to the walls of blood vessels and cause asteriosclerosis, which can lead to coronary heart disease, cerebrovascular disease, or peripheral vascular obstruction. (E. Falk et al., Circulation 92, 657-671, 1995).

In addition, excessive fat components as described above are accumulated in the liver tissue, which may cause fatty liver.

In the above, fatty liver refers to a state in which the ratio of fat to the weight of the liver exceeds 5%, and may be caused not only by intake of excessive fat components but also by intake of alcohol.

Meanwhile, as a method of reducing the blood lipid concentration, diet therapy, exercise therapy, and medicinal therapy are recommended to suppress the intake of foods containing a lot of cholesterol or saturated fatty acids as well as reduce caloric intake.

However, diet therapy or exercise therapy is difficult to strict management and implementation, and its effectiveness is often limited.

Blood lipid concentration reducing agents including bile acid binding resins, HMG-CoA Reductase Inhibitors, neomycin, cholesterol-lowering drugs such as Probucol, fibric acid derivatives, drugs that lower the triglyceride content such as nicotinic acid have been developed to date and are being utilized as therapeutic agents.

However, these drugs have side effects such as hepatotoxicity, gastrointestinal disorders or carcinogenicity. Therefore, research is being conducted on natural products that are safe for humans, have no side effects, and can prevent or treat hyperlipidemia, arteriosclerosis or fatty liver by reducing excessive blood lipid concentration in the blood.

For example, it has been reported that in a case of a hyperlipidemia rat induced by a high fat diet, methanol extract of tissue cultured wild ginseng has the activity of lowering total cholesterol and LDL-cholesterol levels and raising HDL-cholesterol levels, and the petroleum ether extract of ginseng and panaxydol, a component contained in the extract, inhibit cholesterol absorption. In addition, shiitake, ganoderma lucidum, and oyster mushroom powders have been reported to have a cholesterol-lowering effect.

Under this background, the present inventors have made intensive research efforts to develop a food composition having functional properties including improved blood circulation, improved blood triglycerides, improved blood cholesterol, increased tryptophan, and antioxidative activity. As a result, it was confirmed that an anchovy processed product exhibits the above-described improved blood circulation, improved blood triglycerides, improved blood cholesterol, increased tryptophan, and antioxidative activity.

### [Summary of Invention]

### [Problems to be Solved by Invention]

In consideration of the above-mentioned circumstances, it is an object of the present invention to provide a functional food composition using an anchovy processed product as an effective ingredient which has functional properties including improved blood circulation, improved blood triglycerides, improved blood cholesterol, increased tryptophan and antioxidative activity, and a method of manufacturing the same.

In addition, by using natural fish resources, it is another object to provide a functional food composition using an anchovy processed product and a method of manufacturing the same, which can exhibit the above-described effects without side effects such as liver toxicity, gastrointestinal disorders or carcinogenicity.

In addition, it is another object to provide a functional food composition using an anchovy processed product and a method of manufacturing the same, which can improve national health and reduce social and economic expenditures resulting from the above effects by being added as a composition of various seasonings, confectionery, beverages or various health functional foods.

Meanwhile, the objects of the present invention are not limited to the above-mentioned objects, and other objects that are not mentioned will be clearly understood by those skilled in the art from the following description.

### [Means for Solving Problems]

To achieve the objects, according to an aspect of the present invention, semi-dried anchovies semi-dried to a certain moisture content or less, raw anchovies, fermented fish meal-containing soybeans, strains, sea salt, and salted anchovy sauce may be mixed in a predetermined ratio.

Preferably, the functional food composition may be mixed in a ratio of 30% by weight of semi-dried anchovies, 35% by weight of raw anchovies, 10% by weight of fermented fish meal-containing soybeans, 5% by weight of sea salt, and 20% by weight of salted anchovy sauce.

Preferably, the semi-dried anchovies may be semi-dried to a moisture content of 55% or less.

Preferably, the fermented fish meal-containing soybean includes 20% by weight of fish meal added thereto during forming fermented soybeans based on a total weight of the fermented soybeans, and the fish meal is anchovy powders.

Preferably, the strain is Bellegensys (Bacillus velezensis) L2, and is introduced during molding fermented soybeans.

Preferably, the functional food composition comprises an anchovy processed product as an active ingredient and has functional properties including improved blood circulation, improved blood triglycerides, improved blood cholesterol, increased tryptophan, and antioxidative activity.

In addition, in order to solve the above-described problems, a method of manufacturing a functional food composition using an anchovy processed product of the present invention comprises the steps of: preparing a raw material food composition including semi-dried anchovies and raw anchovies; salting the raw material food composition; fermenting the salted food composition under a certain condition; extracting effective ingredients from the fermented food composition; purifying the extracted effective ingredients; and freeze-drying the purified effective ingredients.

Preferably, the raw material food composition is prepared by mixing 30% by weight of semi-dried anchovies, 35% by weight of raw anchovies, 10% by weight of fermented fish meal-containing soybeans, 5% by weight of sea salt, and 20% by weight of salted anchovy sauce with each other.

Preferably, the fermented fish meal-containing soybean is prepared by adding less than 20% by weight of fish meal during forming fermented soybeans based on a total weight of the fermented soybeans, and the fish meal is anchovy powders.

Preferably, the strain is Bellegensys (Bacillus velezensis) L2, and is introduced during molding fermented soybeans.

Preferably, the step of salting the raw material food composition is performed in a salinity of 5±0.1% and the step of fermenting of the salted food composition under a certain condition is performed under an anaerobic condition for 8 weeks at pH of 5.0 to 6.0, 50±1°C, and a stirring speed of 120 RPM.

Preferably, in the step of freeze-drying, the purified effective ingredients are freeze-dried to a moisture content of 10% or less.

Preferably, after the step of freeze-drying the purified effective ingredients, the method of manufacturing a functional food composition using an anchovy processed product may further comprise a sterilization step, a formulation step, a functional food packaging step, a storage step, and a shipping step.

Preferably, the functional food composition includes an anchovy processed product as an effective ingredient, and has functional properties including improved blood circulation, improved blood triglycerides, improved blood cholesterol, increased tryptophan, and antioxidative activity.

Preferably, the salted anchovy sauce is prepared by comprising: a filtrate obtaining step of filtering a fermentation product which is produced through the raw material preparation step of preparing anchovy raw material, and the fermentation step of fermenting the prepared raw material; and removing a residue to obtain a filtrate, wherein the fermentation step is performed by stirring the anchovy raw material at 50 rpm to 300 rpm under an anaerobic condition, the filtrate contains tryptophan among amino acids in an amount of exceeding 5% by weight based on a total amount of the amino acids, and an oxygen partial pressure in the anaerobic condition is equal to or less than that in the atmosphere.

Meanwhile, a method of manufacturing a salted fish sauce with increased tryptophan according to another embodiment of the present invention comprises: a fish and shellfish raw material preparation step of preparing a fish and shellfish raw material; a fermentation step of fermenting the prepared fish and shellfish raw material; and a filtrate obtaining step of filtering a fermented product produced through the fermentation step, and removing a residue to obtain a filtrate, wherein the fermentation step is performed by stirring the raw material at 50 rpm to 300 rpm under an anaerobic condition, the filtrate contains tryptophan among amino acids in an amount of exceeding 5% by weight based on a total amount of the amino acids, and an oxygen partial pressure in the anaerobic condition is equal to or less than that in the atmosphere.

### [Advantageous Effects]

According to an embodiment of the present invention, a functional food composition using an anchovy processed product and a method of manufacturing the same have functional properties including improved blood circulation, improved blood triglycerides, improved blood cholesterol, increased tryptophan, and antioxidative activity.

In addition, by using natural fish resources, the functional food composition according to the present invention can exhibit the above-described effects without side effects such as liver toxicity, gastrointestinal disorders or carcinogenicity.

In addition, the functional food composition according to the present invention can improve national health and reduce social and economic expenditures resulting from the above effects by being added as a composition of various seasonings, confectionery, beverages or various health functional foods.

### [Brief Description of Drawings]

FIG. 1 is an overall process diagram of a method of manufacturing a functional food composition using an anchovy processed product according to an embodiment of the present invention.
FIG. 2 is a graph showing the results of evaluating blood coagulation inhibitory activity related to improved blood circulation of the present invention.
FIG. 3 is a step diagram illustrating a method of manufacturing salted anchovy sauce having increased tryptophan according to an embodiment of the present invention.
FIG. 4 is a graph showing fermentation efficiency depending on a stirring speed in a fermentation step.
FIG. 5 is a graph showing the total nitrogen content of the salted anchovy sauce obtained according to a fermentation time at a different temperature.
FIG. 6 is a graph showing the amino nitrogen content of the salted anchovy sauce obtained according to a fermentation time at a different temperature.
FIG. 7 is a graph showing the histamine content of the salted anchovy sauce obtained according to a fermentation time at a different temperature.
FIG. 8 is a graph showing the volatile basic nitrogen (VBN) content of the salted anchovy sauce obtained according to a fermentation time at a different temperature.
FIG. 9 is a graph showing the pH of the salted anchovy sauce obtained according to a fermentation time at a different temperature.
FIG. 10 is a graph showing the results of the DPPH experiment on the salted anchovy sauce prepared by the method of manufacturing the salted anchovy sauce having increased tryptophan according to an embodiment of the present invention.
FIG. 11 is an image showing the results of the microbial contamination experiment on the salted anchovy sauce prepared by the method of manufacturing the salted anchovy sauce having increased tryptophan according to an embodiment of the present invention.

### [Best mode for carrying out the invention]

The best mode for carrying out the present invention is characterized in that it comprises the steps of: preparing a raw food composition including semi-dried anchovies and raw anchovies; salting the raw food composition; fermenting the salted food composition under a certain condition; extracting effective ingredients from the fermented food composition; purifying the extracted effective ingredients; and freeze-drying the purified effective ingredients.

### [Mode for Carrying out Invention]

As for the terms used in the present invention, general terms that are currently widely used are selected, but in certain cases, some terms are arbitrarily selected by the applicant. In this case, the meaning should be grasped in consideration of the meaning described or used in the specific content for carrying out the invention, not the name of a simple term.

Hereinafter, the technical configuration of the present invention will be described in detail with reference to preferred embodiments of the present invention.

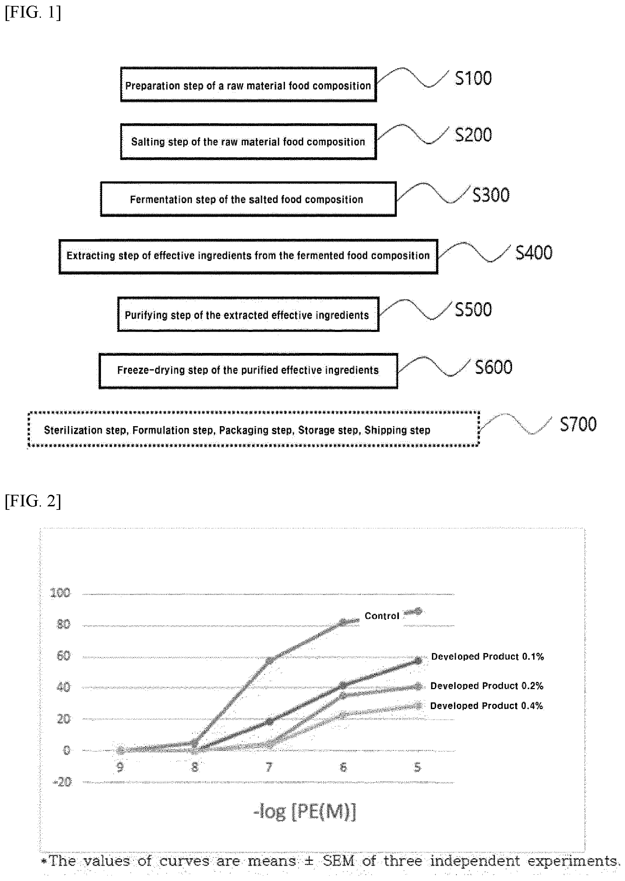

In this regard, FIG. 1 is an overall process diagram of a method of manufacturing a functional food composition using an anchovy processed product according to an embodiment of the present invention, and FIG. 2 is a graph showing the results of evaluating blood coagulation inhibitory activity related to improved blood circulation of the present invention.

Meanwhile, since the functional food composition using an anchovy processed product according to an embodiment of the present invention is the same as the raw material food composition included in a method of manufacturing a functional food composition using an anchovy processed product, hereinafter, the present invention will be described in detail with a focus on the method of manufacturing a functional food composition using an anchovy processed product, and there is no particular limitation on the kind of food in the functional food composition of the present invention, but examples of food include drinks, meat, sausage, bread, Biscuits, rice cakes, chocolates, candies, snacks, sweets, pizza, ramen, other noodles, gums, dairy products including ice cream, various soups, beverages, alcoholic beverages and vitamin complexes, dairy and dairy products, etc. and it includes all health functional foods or health supplements in the usual sense.

Meanwhile, a method of manufacturing a functional food composition using an anchovy processed product according to an embodiment of the present invention includes a preparation step of preparing a raw material food composition including semi-dried anchovies and raw anchovies (S100).

At this time, the raw material food composition consists of semi-dried anchovies semi-dried to a certain moisture content or less, raw anchovies, fermented fish meal-containing soybeans, strains, sea salt and salted anchovy sauce, which are mixed in a predetermined ratio, wherein the functional food composition comprises 30% by weight of semi-dried anchovies, 35% by weight of raw anchovies, 10% by weight of fermented fish meal-containing soybeans, 5% by weight of sea salt, and 20% by weight of salted anchovy sauce, which are mixed with each other.

In addition, the semi-dried anchovies are semi-dried to a moisture content of 55% or less.

Meanwhile, the fermented fish meal-containing soybean is a substance added during forming fermented soybeans, which includes 20% by weight of fish meal added thereto based on a total weight of the fermented soybeans.

At this time, a variety of fish powders may be used as the fish meal but in an embodiment of the present invention, anchovy powders with a certain particle size is used.

Meanwhile, the strain is added during forming fermented soybeans and is involved in the fermentation of a functional food composition. In one embodiment of the present invention, Bacillus velezensis L2 is used as the strain.

At this time, by using the Bacillus velezensis L2, the strain has about 1.5 times higher proteolytic activity and can significantly shorten the fermentation time. Moreover, the content of histamine, which is a component involved in allergic reactions or inflammation, and the content of volatile basic nitrogen, which is an indicator of freshness, are significantly reduced to produce a high-quality functional food.

In addition, a method of manufacturing a functional food composition using an anchovy processed product according to an embodiment of the present invention includes 20% by weight of salted anchovy sauce as a raw material food composition.

At this time, the salted anchovy sauce may be prepared through various manufacturing methods, but the method of manufacturing the salted anchovy sauce according to an embodiment of the present invention comprises: a fish and shellfish raw material preparation step of preparing a fish and shellfish raw material; a fermentation step of fermenting the prepared fish and shellfish raw material; and a filtrate obtaining step of filtering a fermented product produced through the fermentation step, and removing a residue to obtain a filtrate.

At this time, the fish and shellfish raw material means raw anchovies, wherein the fermentation step is performed by stirring the raw material at 50 rpm to 300 rpm under an anaerobic condition, and the filtrate contains tryptophan among amino acids in an amount of exceeding 5% by weight based on a total amount of the amino acids.

At this time, an oxygen partial pressure in the anaerobic condition is equal to or less than that in the atmosphere.

Meanwhile, the salted anchovy sauce by the above-described method is prepared by fermenting the raw anchovies while stirring and then adding salt, so that a fermentation time can be dramatically shortened, and production efficiency can be increased.

In addition, by adding low salinity salt after the fermentation step, it is possible to improve health by reducing the salt content of the product. Furthermore, it is possible to increase antioxidative activity by increasing the content of tryptophan in the salted anchovy sauce.

In addition, by semi-drying the raw anchovies and then completing the fermentation process in a short period of time without adding salt, the content of biogenic amines that may be produced during the fermentation process can be minimized, and the total content of free amino acids is greatly increased. In addition, since the sweet amino acids, the umami amino acids and the bitter amino acids are contained in an optimal ratio, even if tryptophan, which is known as one of the bitter amino acids, is increased significantly, there is an advantage of improving the taste through the interaction of taste.

Meanwhile, a method of manufacturing a functional food composition using an anchovy processed product according to an embodiment of the present invention includes a step of salting the raw material food composition (S200) and a step of fermenting the salted food composition under a certain condition (S300).

In this case, the salting step (S200) is characterized in that a salinity is 5±0.1%, and the fermentation step (S300) may be performed under various conditions, but in an embodiment of the present invention, it is performed under an anaerobic condition for 8 weeks at pH of 5.0 to 6.0, 50±1°C, and a stirring speed of 120 RPM.

Meanwhile, the method of manufacturing a functional food composition using an anchovy processed product according to an embodiment of the present invention includes a step of extracting effective ingredients from the fermented food composition (S400), a step of purifying the extracted effective ingredients (S500), and a step of freeze-drying the purified effective ingredients (S600).

At the freeze-drying step (S600), the purified effective ingredients are freeze-dried to a moisture content of 10% or less.

Meanwhile, after the freeze-drying step (S600), the method of manufacturing a functional food composition using anchovy processed product according to an embodiment of the present invention may further comprise a sterilization step, a formulation step, a functional food packaging step, a storage step, and a shipping step (S700).

At this time, the formulation step is a step of specifying the shape of a functional food composition, and in one embodiment of the present invention, it is formulated as a tablet.

Meanwhile, the functional food composition prepared by the method of manufacturing a functional food composition using an anchovy processed product according to an embodiment of the present invention uses an anchovy processed product as an effective ingredient and has functional properties including improved blood circulation, improved blood triglycerides, and improved blood cholesterol. Hereinafter, the above functional properties of a functional food composition using an anchovy processed product prepared according to an embodiment of the present invention will be described in detail.

### 1. Effect of improved blood circulation

### 1) ACE inhibitory activity evaluation

Angiotensin converting enzyme (Kinase peptidyldipeptide hydrolase, EC 3.4.15.1) cleaves the C-terminal dipeptide (His-Leu) of angiotensin, an inactive decapeptide. Thereby, it plays a role of raising blood pressure by generating angiotensin, an octapeptide that exhibits a strong blood pressure-increasing action by the action of contraction of smooth muscle in the blood vessel wall, and by inactivating bradykinin, a nonapeptide having a strong vasodilating action.

In the mechanism of high blood pressure, the renin-angiotensin system plays a very important role in blood pressure regulation.

In particular, ACE is an enzyme involved in the final step of synthesizing angiotensin, an octapeptide that acts as a vasoconstrictor by hydrolyzing the C-terminal dipeptide from the decapeptide angiotensin generated by renin.

The generated angiotensin promotes the secretion of aldosterone in the adrenal cortex, inhibits the excretion of water and sodium, and suppresses bradykinin, a nonapeptide that has a vasodilating effect, thereby consequently increasing blood pressure.

Therefore, the ACE inhibitory activity prevents vasoconstriction and water retention in the body, thereby lowering blood pressure and improving blood circulation.

**[Table 1] IC50(Half maximal inhibitory concentration) Values of ACE inhibition by salted anchovy sauce**

| Samples | IC50 (ug/mL) |
|---|---|
| Developed product | 392.15±2.20 |
| Control (salted anchovy sauce) | 556.21±3.38 |
| Control (soy sauce) | 713.58±2.26 |

| | |
|---|---|
| *Values are expressed as the mean ±SD (n=3) | |

Referring to Table 1 above, when compared to general salted anchovy sauce and soy sauce using fermented soybeans as a control, the IC50 of a functional food composition (developed product) prepared according to an embodiment of the present invention exhibited a remarkably low value, indicating that the above-described vasoconstriction prevention and thus effect of improved blood circulation were excellent.

### 2) Evaluation of vasoconstriction/relaxation of blood vessel

In this regard, the descending thoracic aorta of a rat was enucleated to evaluate the vasoconstriction/relaxation response of blood vessel by the developed product according to the present invention in an organ bath system.

At this time, the vasoconstriction of blood vessel utilized phenylephrine to determine the degree of vasoconstriction caused by the cumulative dose, the relaxation of blood vessel was induced by inducing the same degree of vasoconstriction in the blood vessels of each treatment group with phenylephrine, and then adding acetylcholine, and the relaxation result was calculated as the relaxation rate for vasoconstriction of phenylephrine and compared with a control.

At this time, the control means a state in which a functional food according to the present invention is not added.

In this regard, referring to FIG. 2, it was confirmed that when the developed product according to the present invention was added, vasoconstriction was inhibited in a concentration-dependent manner compared to the control.

### 2. Effect of improved blood triglycerides

In order to demonstrate the effect of improved blood triglycerides, Tributyrin was utilized to analyze triglyceride inhibitory ability, and more specifically, 1% tributryn solution was diluted to an appropriate amount to prepare a tributyrin plate in a final volume of 5 ml with 2% agar solution. 3 mm diameter well was made on a tributyrin plate, and a functional food composition (developed product) according to the present invention was reacted at 37°C.

A control using general salted anchovy sauce and soy sauce with fermented soybeans were selected and reacted under a same condition.

After the reaction, the size of the clear zone was measured to analyze the lipolytic activity of the sample, and the analysis results are shown in Table 2 below.

**[Table 2]**

| Sample | Control (salted anchovy sauce) | Control (soy sauce) | Developed product |
|---|---|---|---|
| clear zone (mm ²) | 2.06 | 3.01 | 10.25 |

Referring to the Table 2 above, it can be seen that the developed product according to the present invention exhibits the highest triglyceride inhibitory ability.

### 3. Effect of improved blood cholesterol

HMG-CoA reductase inhibitory efficacy was evaluated to demonstrate the effect of improved blood cholesterol. At this time, HMG-CoA reductase inhibitory efficacy was slightly modified from Perchellet's assay (Int. J. Mol. Med., 2009, 24, 633). A sample for detection was dissolved in dimethyl sulfoxide (DMSO) and utilized.

That is, a sample for detection was added to 100 mM NaCl, 1 mM EDTA, 10 mM DTT, 10 mM NADPH, and 100 mM sodium phosphate buffer (pH 6.8) to a 96-well plate, and 10.2 µg/ml of HMG-CoA reductase (8 mM, final concentration) was added to start the reaction.

The activity of HMG-CoA reductase was compared and analyzed by recording the absorbance at 340 nm for 10 minutes at 37°C with a VERSA Max™ microplate reader.

As a control, commercially available salted anchovy sauce and soy sauce using regular fermented soybeans were used, and the experiment was repeated three times.

**[Table 3]**

| Sample | HMG-CoA | IC50(*µ*g/mℓ) |
|---|---|---|
| Control (salted anchovy sauce) | 40.8±2.0 | 152.5±24.8 |
| Control (soy sauce) | 32.3±1.5 | 173.3±17.6 |
| Developed product | 71.8±1.4 | 24.8±0.9 |

Referring to Table 3, the HMG-CoA reductase inhibitory efficacy of the developed product was found to be 6 to 7 times superior to that of the control, and thus it was found to have an excellent effect on lowering the concentration of blood cholesterol.

As a result, a functional food composition using an anchovy processed product according to an embodiment of the present invention and a method of manufacturing the same have improved blood circulation, improved blood triglycerides, improved blood cholesterol, increased tryptophan, and antioxidative activity through the above-described technical configurations.

In addition, by using natural fish resources, the above-described effects can be exhibited without side effects such as liver toxicity, gastrointestinal disorders or carcinogenicity.

In addition, it is added as a composition of various seasonings, confectionery, beverages, or various health functional foods to exert the above-described effects, thereby improving national health and reducing social and economic expenditures.

Hereinafter, a method of manufacturing a salted fish sauce with increased tryptophan according to another embodiment of the present invention will be described in detail.

In this regard, the method of manufacturing a salted fish sauce with increased tryptophan according to an embodiment of the present invention is to prepare a salted fish sauce with increased tryptophan as an effective ingredient through a fermentation process using fish and shellfish as a raw material, by which the content of tryptophan is increased.

The tryptophan is preferably contained in excess of 5% by weight based on the total amount of amino acids.

This increases the content of tryptophan of the amino acids contained in a salted fish sauce, thereby having the advantage that antioxidative activity can be significantly improved compared with conventional salted fish sauces.

Meanwhile, the fish and shellfish can be any one or a mixture of two or more of anchovy, octopus, squid, sardines, cuttlefish, herring, mackerel, mackerel, smelt, canary, yellowtail, skates, tuna, horse mackerel, saury, goggles, badorachi, gulbi, pollock, yangmiri, mussels, barnyards, shrimp and crab, and is not limited thereto as long as it is a raw material that can be used as a raw material for a salted fish sauce.

In addition, based on the total amount of amino acids, it is preferable to contain 35% to 45% by weight of sweet amino acids and 15% to 25% by weight of umami amino acids.

When the content of tryptophan, a bitter-based amino acid, is increased, the content of sweet-based amino acid, content of umami-based amino acid is increased in order to weaken the bitterness by the interaction of taste, thereby improving the taste preference and taste harmony.

Here, the sweet-based amino acids include serine, threonine, glycine, proline, leucine, and allinine, and the umami-based amino acids include glutamic acid and aspartic acid.

In addition, the method of manufacturing a salted fish sauce with increased tryptophan according to the present invention contains a salt content of less than 10% by weight based on the total amount of a salted fish sauce.

This minimizes the salt content in a salted fish sauce, thereby reducing the amount of sodium intake by consumers, thereby having the advantage of improving health.

In addition, the method of manufacturing a salted fish sauce with increased tryptophan according to the present invention contains a histamine content of less than 50 ppm.

The histamine is a kind of biogenic amine, is produced during the fermentation process of protein-containing foods, and causes various side effects in the human body. According to the method of manufacturing a salted fish sauce with increased tryptophan of the present invention, the histamine is contained less than 50 ppm, enabling providing a high-quality salted fish sauce which prevents various side effects in the human body to improve health.

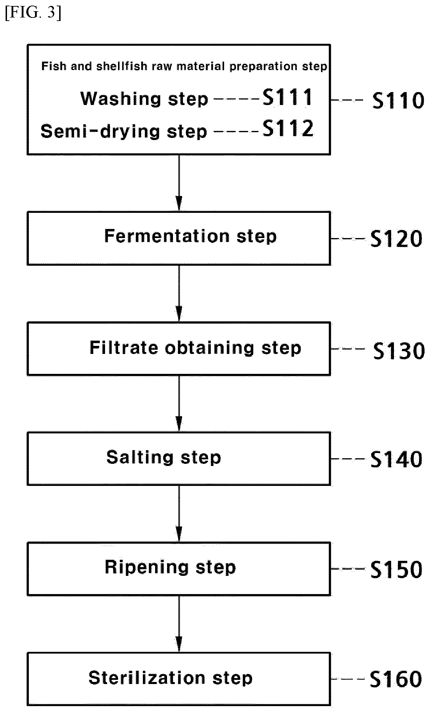

Meanwhile, referring to FIG. 3, which is a step diagram for explaining a method of manufacturing a salted fish sauce with increased tryptophan according to the present invention, the method of manufacturing a salted fish sauce with increased tryptophan according to an embodiment of the present invention relates to a method of manufacturing a salted fish sauce by which a fermentation process is completed in a short period of time by using fish and shellfish as a raw material. Firstly, a fish and shellfish raw material preparation step (S110) of preparing a fish and shellfish raw material is performed.

Here, the fish and shellfish raw material is any one or a mixture of two or more of anchovy, octopus, squid, sardines, cuttlefish, herring, mackerel, mackerel, smelt, canary, yellowtail, skates, tuna, horse mackerel, saury, goggles, badorachi, gulbi, pollock, yangmiri, mussels, barnyards, shrimp and crab, and is not limited thereto as long as it is a raw material that can be used as a raw material for a salted fish sauce.

In addition, the fish and shellfish raw material preparation step of preparing a fish and shellfish raw material (S110) may be performed through a washing step (S111) and a semi-drying step (S112).

In more detail, the washing step (S111) is a process of sufficiently washing the fish and shellfish raw material with brine or water, and the semi-drying step (S112) is a process of drying the raw material of the washed fish and shellfish to an appropriate moisture content.

At this time, the drying means for drying the fish and shellfish raw material may be a conventional dryer, but is not limited thereto.

In addition, in the semi-drying step (S112), the fish and shellfish raw material is preferably dried to have a moisture content of 30% to 55%.

The reason is that when the moisture content is less than 30%, the fermentation efficiency in a fermentation step to be described later decreases, and when the moisture content exceeds 55%, the amount of biogenic amines produced in the fermentation step is increased. Or worse, it proceeds to spoilage.

Next, a fermentation step (S120) of fermenting the prepared fish and shellfish raw material is performed.

At this time, it is preferable that no separate water is added to the fish and shellfish raw material, and that only fish and shellfish raw material having a moisture content of 30% to 55% through the semi-drying step (S112) is introduced into the fermentor.

The fermentation step (S120) is performed while stirring the prepared fish and shellfish raw material under an anaerobic condition.

In this case, the stirring speed is preferably 50 rpm to 300 rpm.

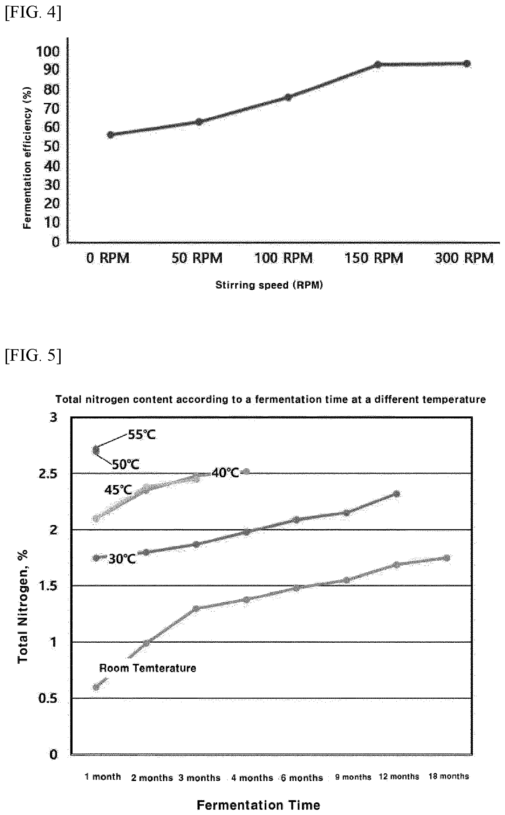

The reason is to maximize the fermentation efficiency of the fermentation step, as shown in FIG. 4. When the stirring rpm is less than 50 rpm, the effect of increasing the fermentation efficiency is insignificant, and when the stirring rpm exceeds 300 rpm, the fermentation efficiency is no longer increased.

Meanwhile, the anaerobic condition according to the present invention is a "facultative anaerobic" condition, and the facultative anaerobic condition means that the oxygen partial pressure is equal to or less than that in the atmosphere, and more specifically, the oxygen concentration is 2 to 10%.

Meanwhile, in the present invention, the "fermentation efficiency" was calculated as "total nitrogen content of the obtained salted fish sauce / total nitrogen content of the injected fish and shellfish raw material X 100".

In addition, the fermentation temperature in the fermentation step (S120) is preferably processed at a constant temperature in a range of 45 °C to 55 °C.

The reason is that when the fermentation temperature is less than 45° C., the fermentation time is increased, and when the fermentation temperature exceeds 55° C., the fermentation time is no longer shortened and protein modification may occur during the fermentation process.

In the method of manufacturing a salted fish sauce with increased tryptophan according to the present invention, the fermentation step proceeds under an optimal stirring condition and an optimal temperature condition without adding salt, so that the fermentation process is completed within 7 days, compared to the conventional technology. Therefore, it has the advantage of maximizing the production efficiency of a salted fish sauce by remarkably shortening the fermentation time, and further, it has the advantage of minimizing the production of biogenic amines by shortening the fermentation time.

Next, a filtrate obtaining step (S130) of filtering a fermentation product which is produced through the fermentation step of fermenting the prepared raw material, and removing a residue to obtain a filtrate is performed.

Thereafter, a salting step (S140) of salting the filtrate is performed.

Here, the salt is added in less than 10% by weight of salt based on 100% by weight of the fish and shellfish raw material.

This minimizes the amount of salt contained in a salted fish sauce, thereby reducing the amount of sodium intake of consumers, thereby having the advantage of improving health.

In addition, it is preferable that the salt is sea salt.

In addition, in the method of manufacturing a salted fish sauce with increased tryptophan according to the present invention, after the salting step (S140), a ripening step (S150) and a sterilization step (S160) may be further sequentially performed.

The ripening step (S150) is a process of ripening the filtrate to which salt is added, and is ripened for 10 to 15 days at a constant temperature of 45°C to 55°C.

In addition, the sterilization step (S160) is a process of obtaining a liquid salted fish sauce by sterilizing the ripened filtrate, and heat treatment is performed to the ripened filtrate for 5 minutes to 30 minutes at a constant temperature of 70°C to 100°C to obtain a salted fish sauce.

The salted fish sauce prepared by the method of manufacturing a salted fish sauce with increased tryptophan contains tryptophan among amino acids in excess of 5% by weight based on the total amount of amino acids.

This has the advantage that the content of tryptophan among the amino acids contained in the salted fish sauce is significantly increased, so that the antioxidative activity can be greatly improved compared to the conventional salted fish sauce.

In addition, based on the total amount of amino acids, the salted fish sauce contains 35% to 45% by weight of sweet-based amino acids and 15 to 25% by weight of umami-based amino acids.

The content of tryptophan, which is an amino acid of the bitter taste, is increased, but the content of sweet-based amino acids and the content of umami-based amino acids are increased together, so that the bitterness component is weakly felt by the interaction of taste, so that the taste preference and taste harmony can be increased.

Here, the sweet-based amino acids include serine, threonine, glycine, proline, leucine, and allinine, and the umami-based amino acids may include glutamic acid and aspartic acid.

In addition, the salted fish sauce contains less than 50 ppm of histamine, a kind of biogenic amine.

This gets the histamine, which is known to cause various side effects in the human body, to be contained in an amount of less than 50 ppm, thereby preventing various side effects in the human body, thereby having the benefit of improving health.

### (Example 1)

A raw material was prepared by washing twice with brine using fresh anchovies with a length of about 17 cm caught in the south coast as a fish and shellfish raw material, and then semi-dried to a moisture content of 30% to 55%.

Thereafter, the semi-dried anchovies were fermented while being stirred at 50 rpm at a temperature of 50°C under an anaerobic condition to produce a fermented product.

At this time, the fermentation time was completed in 48 hours.

Thereafter, the resulting fermented product was filtered to remove a residue, and a filtrate was obtained, and then less than 10% by weight of salt was added based on 100& by weight of the raw material to obtain a salted fish sauce (Example 1).

### (Example 2)

Compared with Example 1, a salted fish sauce (Example 2) was obtained in the same manner as the rest of the process, except that a stirring rpm was applied at 100 rpm.

### (Example 3)

Compared with Example 1, except that a stirring rpm was applied at 150 rpm, the rest of the processes were the same to obtain a salted fish sauce (Example 3).

### (Example 4)

Compared with Example 1, except that a stirring rpm was applied at 300 rpm, the rest of the process was the same to obtain a salted fish sauce (Example 4).

### (Comparative Example 1)

After washing twice using fresh anchovies with a length of about 17 cm caught in the same southern coast as in the examples as a fish and shellfish raw material, 20% by weight of salt was added based on 100% by weight of the washed raw material, and fermentation was performed at 30°C and under an anaerobic condition to produce a fermented product.

At this time, the fermentation proceeded for 12 months.

Thereafter, the produced fermented product was filtered to remove a residue, and a salted fish sauce (Comparative Example 1) was obtained.

### (Comparative Examples 2 to 4)

Salted fish sauce A, salted fish sauce B, and salted fish sauce C distributed in Korea were used as a Comparative Example 2, a Comparative Example 3, and a Comparative Example 4, respectively.

### (Experimental Example 1: Analysis of fermentation quality by a stirring rpm)

Using a Comparative Example 1 and Examples 1 to 4, total nitrogen content, amino nitrogen content, histamine content, volatile basic nitrogen (VBN, Volatile Basic Nitrogen) content, and PH were confirmed, and the results are shown in Table 4 and Figure 4 below.

**[Table 4]**

| | Comparative Example 1 | Example 1 | Example 2 | Example 3 | Example 4 |
|---|---|---|---|---|---|
| Total nitrogen content (%) | 1.7 | 1.9 | 2.25 | 2.8 | 2.78 |
| Amino nitrogen content (AN, mg/100g) | 879 | 1,128 | 1,521 | 2,670 | 2,670 |
| Histamine content (ppm) | 658 | 359 | 40 | 12 | 15 |
| Volatile basic nitrogen content (mg%) | 320 | 258 | 110 | 40 | 45 |
| PH | 6 | 5.8 | 5.6 | 5.2 | 5.22 |

That is, as shown in Table 4 and FIG. 4, compared to Comparative Example 1, Examples 1 to 4 have a higher content of total nitrogen and amino nitrogen, and less content of histamine, one of the biogenic components, and the production amount of volatile basic nitrogen is low. So, it is prepared with an excellent salted fish sauce.

In addition, when the pH of a salted fish sauce exceeds 5.8, gallstone-type precipitates may be generated during distribution and storage, but Examples 1 to 5 are prepared with a pH of 5.8 or less, thereby having the advantage of not generating such gallstone-type precipitates.

In particular, in the case of Example 3 stirred at 150 rpm, it was confirmed that it was prepared with the best salted fish sauce in all quality indicators including total nitrogen, amino nitrogen, histamine content, volatile basic nitrogen, and PH.

That is, in the fermentation step, the stirring speed is preferably between 50 rpm and 300 rpm, because when the stirring rpm is less than 50 rpm, the content of total nitrogen and amino nitrogen decreases. When the stirring rpm exceeds 300 rpm, the generation amount of histamine, which is one of the components of the biogenic amine, is increased, the generation amount of volatile base nitrogen is low, and the PH is increased, so that gallstone precipitates may be generated. This is because when the stirring rpm exceeds 300 rpm, the fermentation efficiency is no longer increased.

### (Experimental Example 2: Analysis of fermentation quality by temperature conditions)

Compared with Example 3, the fermentation temperature conditions in the fermentation step were applied in the same manner, except for fermentation at a room temperature, 30°C, 40°C, 45°C, 50°C, and 55°C, respectively.
* Total nitrogen content, amino nitrogen content, histamine content, volatile basic nitrogen (VBN, Volatile Basic Nitrogen) content, and PH of the salted fish sauce obtained according to the fermentation time at different temperature conditions were respectively confirmed, and the results are shown in Tables 5 to 9 below, and are graphed in FIGS. 5 to 9.

**[Table 5]**

| Total nitrogen content | Room temperature | 30°C | 40°C | 45°C | 50°C | 55°C |
|---|---|---|---|---|---|---|
| 1 month | 0.6 | 1.75 | 2.1 | 2.1 | 2.7 | 2.72 |
| 2 month | 0.99 | 1.8 | 2.35 | 2.38 | - | - |
| 3 month | 1.3 | 1.87 | 2.48 | 2.45 | - | - |
| 4 month | 1.38 | 1.98 | 2.52 | - | - | - |
| 6 month | 1.48 | 2.09 | - | - | - | - |
| 9 month | 1.55 | 2.15 | - | - | - | - |
| 12 month | 1.69 | 2.32 | - | - | - | - |
| 18 month | 1.75 | - | - | - | - | - |

Referring to Table 5 and FIG. 5, when the fermentation temperature is 50° C., the total nitrogen content is 2.7% within 1 month, and when the fermentation temperature is 55° C., the total nitrogen content is 2.72% within 1 month.

**[Table 6]**

| Amino nitrogen content (mg/100g) | Room temperature | 30°C | 40°C | 45°C | 50°C | 55°C |
|---|---|---|---|---|---|---|
| 1 month | 389 | 1320 | 2524 | 2530 | 2700 | 2701 |
| 2 month | 488 | 1452 | 2588 | 2602 | - | - |
| 3 month | 652 | 1554 | 2648 | 2658 | - | - |
| 4 month | 786 | 1766 | 2680 | - | - | - |
| 6 month | 987 | 2002 | - | - | - | - |
| 9 month | 1157 | 2138 | - | - | - | - |
| 12 month | 1385 | 2320 | - | - | - | - |
| 18 month | 1520 | - | - | - | - | - |

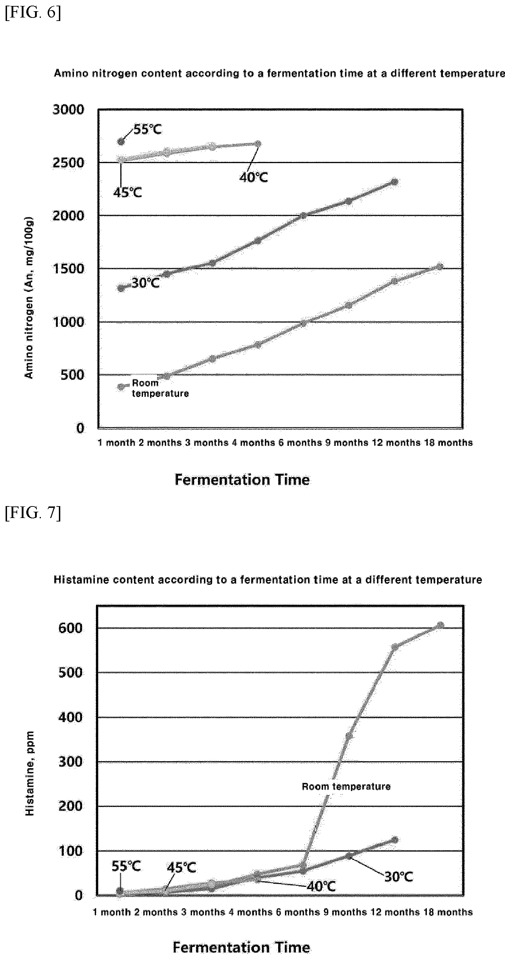

Referring to Table 6 and FIG. 6, the amino nitrogen content (mg/100g) is 2700mg/100g within 1 month when the fermentation temperature is 50°C, and the amino nitrogen content (mg/100g) is 2701mg/100g within 1 month when the fermentation temperature is 55°C. That is, when the fermentation temperature is at a room temperature, it has been confirmed that fermentation is not completed even if the fermentation process proceeds for 18 months, and when the fermentation temperature is 30°C, a fermentation time of 12 months is required. It was confirmed respectively that fermentation was completed within 4 months when the fermentation temperature is 40°C, 3 months when the fermentation temperature is 45°C, and within 1 month when the fermentation temperature is 50°C and 55°C.

**[Table 7]**

| Histamine content(ppm) | Room temperature | 30°C | 40°C | 45°C | 50°C | 55°C |
|---|---|---|---|---|---|---|
| 1 month | 3 | 3 | 6 | 5 | 10 | 11 |
| 2 month | 12 | 7 | 15 | 10 | - | - |
| 3 month | 20 | 15 | 28 | 25 | - | - |
| 4 month | 48 | 40 | 35 | - | - | - |
| 6 month | 68 | 55 | - | - | - | - |
| 9 month | 358 | 89 | - | - | - | - |
| 12 month | 557 | 125 | - | - | - | - |
| 18 month | 606 | - | - | - | - | - |

Referring to Table 7 and FIG. 7, the histamine content (ppm) was confirmed to be 10 ppm when the fermentation temperature is 50°C, 11 ppm when the fermentation temperature is 55°C, and 25 ppm when the fermentation temperature is 50°C. Meanwhile, when the fermentation temperature is at a room temperature, 30°C, or 40°C, it was confirmed that the content of histamine was increased as the fermentation time was increased. Therefore, it was confirmed that the amount of histamine produced can be reduced by reducing the fermentation time.

**[Table 8]**

| VBN content(mg%) | Room temperature | 30°C | 40°C | 45°C | 50°C | 55°C |
|---|---|---|---|---|---|---|
| 1 month | 56 | 52 | 102 | 138 | 224 | 223 |
| 2 month | 89 | 60 | 185 | 157 | - | - |
| 3 month | 114 | 66 | 202 | 198 | - | - |
| 4 month | 138 | 89 | 228 | - | - | - |
| 6 month | 185 | 115 | - | - | - | - |
| 9 month | 220 | 160 | - | - | - | - |
| 12 month | 259 | 210 | - | - | - | - |
| 18 month | 321 | - | - | - | - | - |

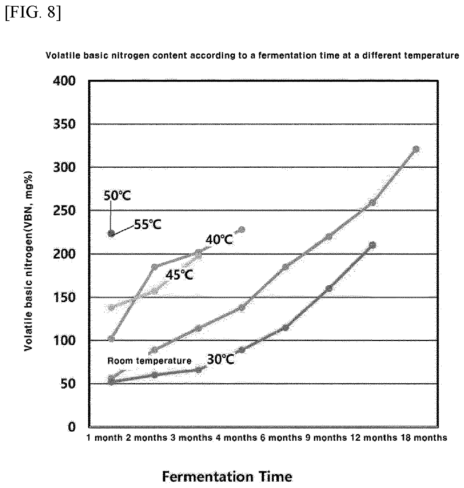

Referring to Table 8 and FIG. 8, when the fermentation temperature is 50°C and 55°C, fermentation is completed within 1 month, and the volatile basic nitrogen content (mg%) was confirmed to be 224mg% and 223mg%, respectively. In addition, fermentation is completed within 3 months when the fermentation temperature is 45°C, the volatile base nitrogen content is 198mg%. Fermentation is completed within 4 months when the fermentation temperature is 40°C, and the volatile base nitrogen content is 228mg%. When the fermentation temperature is 30°C, the fermentation is completed within 12 months and the volatile base nitrogen content is 210mg%. In the case of a room temperature, the fermentation time takes more than 18 months, and the volatile base nitrogen content was confirmed to be 321mg%.

**[Table 9]**

| PH | Room temperature | 30°C | 40°C | 45°C | 50°C | 55°C |
|---|---|---|---|---|---|---|
| 1 month | 6.65 | 6.05 | 5.87 | 5.56 | 5.16 | 5.18 |
| 2 month | 6.59 | 6.02 | 5.55 | 5.44 | - | - |
| 3 month | 6.48 | 5.99 | 5.38 | 5.39 | - | - |
| 4 month | 6.46 | 5.82 | 5.22 | - | - | - |
| 6 month | 6.44 | 5.77 | - | - | - | - |
| 9 month | 6.32 | 5.55 | - | - | - | - |
| 12 month | 6.21 | 5.33 | - | - | - | - |
| 18 month | 6.08 | - | - | - | - | - |

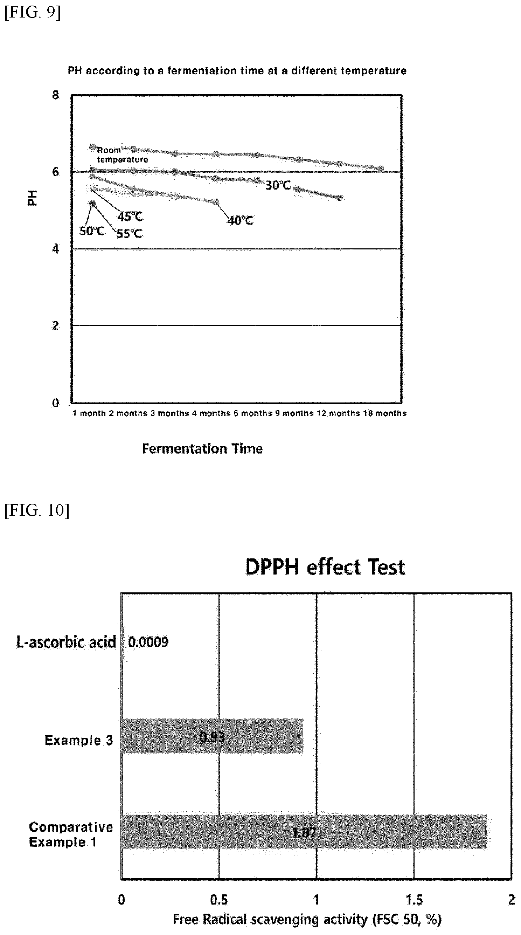

Referring to Table 9 and FIG. 9, when the fermentation temperature is 50°C and 55°C, the fermentation is completed within 1 month, and the pH is confirmed to be 5.16 and 5.18 respectively. In addition, when the fermentation temperature is 45°C, fermentation is completed within 3 months, and the PH is confirmed to be 5.39mg%. When the fermentation temperature is 40°C, the fermentation is completed within 4 months, and the pH is confirmed to be 5.22. When the fermentation temperature is 30°C, fermentation is completed within 12 months, and the pH is confirmed to be 5.33. At a room temperature, the fermentation time takes more than 18 months, and the pH is confirmed to be 6.08.

Meanwhile, when the pH of a salted fish sauce exceeds 5.8, a problem in which gallstone-like precipitates may be generated during the distribution or storage process has been confirmed. Therefore, it is an important technical task to make the pH of a salted fish sauce less than 5.8. The method of manufacturing a salted fish sauce according to the present invention has the advantage that a salted fish sauce can be prepared with a pH of less than 5.8 without adding additional additives.

That is, from an Experimental Example 2, as a result of checking the quality indicators for the total nitrogen content, amino nitrogen content, histamine content, volatile basic nitrogen (VBN, Volatile Basic Nitrogen) content, and PH of the obtained salted fish sauces, the fermentation temperature in the fermentation step was confirmed to be 45°C and 55°C.

### (Experimental Example 3: Analysis of Amino Acid Components)

Example 3 and Comparative Example 1 were prepared and analyzed for free amino acids, and the results are shown in Table 10 below.

Here, free amino acid analysis was performed using an HPLC system.

**[Table 10]**

| Samples | Amino acids | Comparative Example 1(µg/ml) | Proportion of Example 1 | Example 3 (µg/ml) | Proportion of Example 3 |
|---|---|---|---|---|---|
| Sweet-based amino acids | Serine | 136.54 | 2.87% | 113.43 | 2.02% |
| | Threonine | 140.57 | 2.96% | 94.84 | 1.69% |
| | Glycine | 575.55 | 12.11% | 828.89 | 14.78% |
| | Proline | 110.5 | 2.32% | 99.11 | 1.77% |
| | Leucine | 400.48 | 8.42% | 385.93 | 6.88% |
| | Alanine | 493.3 | 10.38% | 648.95 | 11.58% |
| Total | | 1856.94 | 39.06% | 2171.15 | 38.73% |
| Umami-based amino acids | Glutamic acid | 744.93 | 15.67% | 999.87 | 17.83% |
| | Aspartic acid | 224.68 | 4.73% | 223.98 | 4.00% |
| Total | | 969.61 | 20.39% | 1223.85 | 21.83% |
| | Valine | 232.41 | 4.89% | 257.19 | 4.59% |
| Bitter-based amino acids | Isoleucine | 206.39 | 4.34% | 201.57 | 3.60% |
| | Methionine | 196.78 | 4.14% | 220.03 | 3.92% |
| | Phenylalanine | 198.43 | 4.17% | 207.28 | 3.70% |
| | Tyrosine | 53.19 | 1.12% | 33.37 | 0.60% |
| | Lysine | 497.47 | 10.46% | 488.64 | 8.72% |
| | Arginine | 138.71 | 2.92% | 27.45 | 0.49% |
| | Histidine | 259.79 | 5.46% | 285.61 | 5.09% |
| | Tryptophan | 144.5 | 3.04% | 490.16 | 8.74% |
| Total | | 1927.67 | 40.55% | 2211.3 | 39.44% |
| Sum of Total | | 4754.22 | 100% | 5606.3 | 100% |

As shown in Table 10, Example 3 showed the content of tryptophan of 490.16 (8.74%), and in the case of Comparative Example 1, the content of tryptophan was confirmed to be 144.5 (3.04%). Accordingly, it was confirmed that the antioxidative activity of a salted fish sauce prepared by the method of manufacturing a salted fish sauce with increased tryptophan of the present invention can be significantly improved.

In addition, referring to Table 10, in Example 3, it was confirmed that the content of sweet-based amino acids was 2171.15 (38.73%), the content of umami-based amino acids was 1223.85 (21.83%), and the content of bitter-based amino acids was 2211.3 (39.44%). In the case of Comparative Example 1, the content of sweet-based amino acids was 1856.94 (39.06%), the content of umami-based amino acids was 969.61 (20.39%), and the content of bitter-based amino acids was 1927.67 (40.55%).

That is, the salted fish sauce prepared by the method of manufacturing a salted fish sauce with increased tryptophan according to the present invention has a significantly increased content of free amino acids as a whole, compared to the salted fish sauce of Comparative Example 1. Since the amino acid content ratio of the sweet-based amino acids and umami-based amino acids was increased, and the content of bitter-based amino acids was decreased, it was confirmed that it had an effect of improving taste by the interaction of taste.

### (Experimental Example 4: Analysis of salt content)

Example 3 and Comparative Example 1 were prepared, and the salt content was analyzed using a salinity meter, and the results are shown in Table 11 below.

**[Table 11]**

| | Comparative Example 1 | Example 3 |
|---|---|---|
| Salinity (%) | 23% | 8% |

As shown in Table 11, it was confirmed that the salinity of Example 3 was 8% and that of Comparative Example 1 was 23%. That is, compared with the salted fish sauce of the comparative example 1, the salted fish sauce prepared by the method of manufacturing a salted fish sauce with increased tryptophan according to the present invention has a low salinity, thereby providing a salted fish sauce with improved health.

### (Experimental Example 5: Comparison of histamine content)

Comparative Examples 1 to 4, and Example 3 were prepared, and the histamine content was analyzed, and the results are shown in Table 12 below.

**[Table 12]**

| | Comparative Example1 | Comparative Example2 | Comparative Example3 | Comparative Example4 | Example 3 |
|---|---|---|---|---|---|
| Histamine content (PPM) | 520 | 256 | 606 | 579 | 12 |

As shown in Table 12, the histamine content of Comparative Example 1 was 520 ppm, the histamine content of Comparative Example 2 was 256 ppm, the histamine content of Comparative Example 3 was 606 ppm, the histamine content of Comparative Example 4 was 579 ppm, and the histamine content of Example 3 was 12 ppm. In other words, a salted fish sauce prepared by the method of manufacturing a salted fish sauce with increased tryptophan according to the present invention has significantly less histamine content than in the comparative examples, so that it can be provided as a high-quality salted fish sauce with improved health.

### (Experimental Example 6: Analysis of total nitrogen content)

Comparative Examples 1 to 4, and Example 3 were prepared, and the total nitrogen content was analyzed, and the results are shown in Table 13 below.

**[Table 13]**

| | Comparative Example 1 | Comparative Example 2 | Comparative Example 3 | Comparative Example 4 | Example 3 |
|---|---|---|---|---|---|
| Total nitrogen content (TN,%) | 1.7 | 0.6 | 1.2 | 1.6 | 2.8 |

As shown in Table 13, the total nitrogen content of Comparative Example 1 was 1.7%, the total nitrogen content of Comparative Example 2 was 0.6%, the total nitrogen content of Comparative Example 3 was 1.2%, the total nitrogen content of Comparative Example 4 was 1.6%, and the total nitrogen content of Example 3 was 2.8%. That is, compared with the comparative examples, a salted fish sauce prepared by the method of manufacturing a salted fish sauce with increased tryptophan according to the present invention can be provided as a high-quality salted fish sauce because the total nitrogen content is increased.

### (Experimental Example 7: Sensory test)

As an evaluation of the sensory palatability of the salted fish sauce of Example 3 and the salted fish sauce of Comparative Example 1, a sensory test was performed and shown in Table 11 below.

In addition, a sensory test was conducted on leek geotjeolyi prepared using the salted fish sauce of Example 3 and the salted fish sauce of Comparative Example 1, respectively, and are shown in Table 14 below.

Here, the evaluation items were overall preference, sweet, salty, umami, fishy, fishy, bitter, and taste harmony, and a sensory test was conducted on 30 target consumer panels who actually use the product.

**[Table 14]**

| | Overall preference | Sweet taste | Salty taste | Umami taste | Fishy taste | Fishy smell | Bitter taste | Taste harmony |
|---|---|---|---|---|---|---|---|---|
| Comparative Example 1 | 5.0 | 4.8 | 4.2 | 4.8 | 4.4 | 4.0 | 3.5 | 3.8 |
| Example 3 | 6.2 | 6.0 | 5.8 | 6.3 | 2.3 | 2.2 | 2 | 5.8 |

**[Table 15]**

| | Overall preference | Sweet taste | Salty taste | Umami taste | Fishy taste | Fishy smell | Bitter taste | Taste harmony |
|---|---|---|---|---|---|---|---|---|
| Comparative Example 1 | 4.8 | 4.2 | 4.2 | 5.0 | 4.2 | 3.8 | 3.5 | 4.0 |
| Example 3 | 6.3 | 5.8 | 5.8 | 6.2 | 2.2 | 2.4 | 2 | 6.0 |

As a result, as shown in Tables 14 and 15, the salted fish sauce prepared by the method of manufacturing a salted fish sauce with increased tryptophan according to the present invention was found to be superior in overall preference and taste harmony compared to the salted fish sauce of Comparative Example 1. In particular, in the case of tryptophan, which is a bitter amino acid, the salted fish sauce of the comparative example accounts for about 3.04% of the total amino acid weight, whereas the salted fish sauce prepared by the method of manufacturing a salted fish sauce with increased tryptophan according to the present invention is about 8.74% of the total amino acid weight. Even though it occupied a high proportion, it was confirmed that the bitter taste was felt weaker in the evaluation of the sensory palatability of the salted fish sauce and the leek geotjeolyi evaluation.

In addition, it was found that the salted fish sauce prepared by the method of manufacturing a salted fish sauce with increased tryptophan according to the present invention feels very weak compared to the comparative example 1.

### (Experimental Example 8: Analysis of 9 major nutrients)

Comparative Examples 2 to 4, and Example 3 were prepared, and major nutrients were analyzed, and the results are shown in Table 16 below.

**[Table 16]**

| | Comparative Example 2 | Comparative Example 3 | Comparative Example 4 | Example 3 |
|---|---|---|---|---|
| Calories (kcal/100ml) | 31 | 38.2 | 61 | 127.4 |
| Carbohydrates (g/100ml) | 1.6 | 1.8 | 2.9 | 9.7 |
| Sugars (g/100ml) | 0 | 0 | 0 | 2.9 |
| Proteins (g/100ml) | 5.7 | 7.3 | 11.9 | 22.1 |
| Fat (g/100ml) | 0.2 | 0.2 | 0.2 | 0 |
| Saturated fat (g/100ml) | 0 | 0 | 0 | 0 |
| Trans fat (g/100ml) | 0 | 0 | 0 | 0 |
| Cholesterol (mg/100ml) | 0 | 0 | 0 | 0 |

As shown in Table 16, compared to Comparative Examples 2 to 4, in Example 3, it was confirmed that calories, carbohydrates, sugars, and protein components were significantly increased, and fat components were decreased. That is, the salted fish sauce prepared by the method of manufacturing a salted fish sauce with increased tryptophan according to the present invention increases calories, carbohydrates, sugars, and protein components, and reduces fat components, so that it can be provided as a high-quality salted fish sauce.

### (Experimental Example 9: Analysis of antioxidative activity)

In order to confirm the antioxidative activity of Comparative Example 1 and Example 3, the DPPH test method was performed, and the results are shown in FIG. 10.

At this time, in the DPPH test method, purple diphenylpicrylhydrazyl (free radical) reacts with an antioxidative activity and is converted to pale yellow diphenylpicryldyrazine (nonradical). This is a method of evaluating the antioxidative activity through the change in color. The absorbance value (OD) was obtained at 517 nm, which is the maximum absorption wavelength of purple, and the antioxidative activity of L-ascorbic acid, Comparative Examples 1 and 3 was evaluated.

As shown in FIG. 10, the concentration of L-ascorbic acid required to remove 50% of DPPH radicals is 0.0009% (FSC 50 = 0.0009%), and the concentration of Comparative Example 1 required to remove 50% of DPPH radicals is 1.87. % (FSC 50 = 1.87%), the concentration of Example 3 required to remove 50% of DPPH radicals was found to be 0.93% (FSC 50 = 0.93%).

Accordingly, it was confirmed that the salted fish sauce prepared by the method of manufacturing a salted fish sauce with increased tryptophan according to the present invention has an antioxidative activity that is about twice as high as in Comparative Example 1.

### (Experimental Example 10: Analysis of microbial contamination)

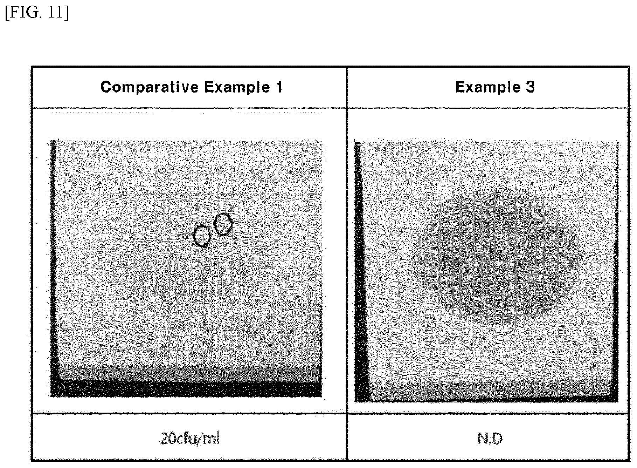

Comparative Example 1 and Example 3 were prepared, and the degree of contamination of common bacteria and microorganisms was measured, and the results are shown in FIG. 11.

Here, microbial contamination analysis was performed by exposing Comparative Examples 1 and 3 to a room temperature for 48 hours, diluting 10 times, inoculating in 3M petrifilm medium, and incubating at 35±1° C. for 48 hours to measure contamination.

As shown in FIG. 11, in the case of Comparative Example 1, as a result of measuring the degree of contamination of common bacteria and microorganisms, it was measured as 20 cfu/ml, and in the case of Example 3, no contaminating bacteria were detected.

It is believed that when Comparative Example 1 was stored at a room temperature, it would show a trend value similar to 27 to 190 cfu/mL, which is a measured value of a commercial fish sauce, and in Example 3, even when stored at a room temperature, contaminants were not detected.

As described above, a method of manufacturing a salted fish sauce with increased tryptophan according to the present invention is prepared by stirring and fermenting fish and shellfish raw material and then adding low salt, so that a fermentation time can be significantly shortened, and it is provided with low salt and has antioxidative activity, thereby having an advantage that can improve health.

Furthermore, since the fermentation process is completed in a short period of time, the content of histamine generated during the fermentation process can be minimized, the total content of free amino acids is greatly increased, and the sweet-based amino acids, umami-based amino acids and bitter-based amino acids are contained in an optimal ratio. As a result, even if the content of tryptophan, which is known as one of the amino acids of the bitter taste, is increased significantly, it has the effect of improving the taste through the interaction of taste.

As described above, the present invention has been illustrated and described with reference to preferred embodiments, but is not limited to the above-described embodiments, and various changes or modifications may be made by those of ordinary skill in the art to which the present invention pertains within the scope not departing from the spirit of the present invention.

### [Industrial availability]

The present invention relates to a functional food composition using an anchovy processed product and a method of manufacturing the same, and uses an effective ingredient included in the anchovy processed product to improve blood circulation, improve blood triglycerides, improve blood cholesterol, increase tryptophan, and have antioxidative activity.

## Claims

1. A functional food composition using an anchovy processed product, comprising semi-dried anchovies dried to a certain moisture content or less, raw anchovies, fermented fish meal-containing soybeans, strains, sea salt and salted anchovy sauce, which are mixed in a predetermined ratio.

2. The functional food composition using an anchovy processed product according to claim 2, wherein the functional food composition comprises 30% by weight of semi-dried anchovies, 35% by weight of raw anchovies, 10% by weight of fermented fish meal-containing soybeans, 5% by weight of sea salt, and 20% by weight of salted anchovy sauce, which are mixed with each other.

3. The functional food composition using an anchovy processed product according to claim 1 or 2, wherein the semi-dried anchovies are semi-dried to a moisture content of 55% or less.

4. The functional food composition using an anchovy processed product according to claim 1 or 2, wherein the fermented fish meal-containing soybean includes 20% by weight of fish meal added thereto during forming fermented soybeans based on a total weight of the fermented soybeans, and the fish meal is anchovy powders.

5. The functional food composition using an anchovy processed product according to claim 1 or 2, wherein the strain is Bacillus velezensis L2, and is introduced during molding fermented soybeans.

6. The functional food composition using an anchovy processed product according to claim 1 or 2, wherein the functional food composition comprises the anchovy processed product as an effective ingredient, and has functional properties including, improved blood triglycerides, improved blood cholesterol, increased tryptophan, and antioxidative activity.

7. A method of manufacturing a functional food using an anchovy processed product comprising the steps of:
preparing a raw material food composition including semi-dried anchovies and raw anchovies;
salting the raw material food composition;
fermenting the salted food composition under a certain condition;
extracting effective ingredients from the fermented food composition;
purifying the extracted effective ingredients; and
freeze-drying the purified effective ingredients.

8. The method of manufacturing a functional food using an anchovy processed product according to claim 7, wherein the raw material food composition is prepared by mixing 30% by weight of semi-dried anchovies, 35% by weight of raw anchovies, 10% by weight of fermented fish meal-containing soybeans, 5% by weight of sea salt, and 20% by weight of salted anchovy sauce with each other.

9. The method of manufacturing a functional food using an anchovy processed product according to claim 7 or 8, wherein the fermented fish meal-containing soybean is prepared by adding 20% by weight of fish meal during forming fermented soybeans based on a total weight of the fermented soybeans, and the fish meal is anchovy powders.

10. The method of manufacturing a functional food using an anchovy processed product according to claim 7 or 8, wherein the strain is Bacillus velezensis L2, and is introduced during molding fermented soybeans.

11. The method of manufacturing a functional food using an anchovy processed product according to claim 7 or 8, wherein the step of salting the raw material food composition is performed in a salinity of 5±0.1%.

12. The method of manufacturing a functional food using an anchovy processed product according to claim 7 or 8, wherein the step of fermenting of the salted food composition under a certain condition is performed under an anaerobic condition for 8 weeks at pH of 5.0 to 6.0, 50±1°C, and a stirring speed of 120 RPM.

13. The method of manufacturing a functional food using an anchovy processed product according to claim 7 or 8, wherein in the step of freeze-drying, the purified effective ingredients are freeze-dried to a moisture content of 10% or less.

14. The method of manufacturing a functional food using an anchovy processed product according to claim 7 or 8, wherein after the step of freeze-drying the purified effective ingredients, further comprising: a sterilization step, a formulation step, a functional food packaging step, a storage step, and a shipping step.

15. The method of manufacturing a functional food using an anchovy processed product according to claim 7 or 8, wherein the functional food includes the anchovy processed product as an effective ingredient, and has functional properties including improved blood circulation, improved blood triglycerides, improved blood cholesterol, increased tryptophan, and antioxidative activity.

16. The method of manufacturing a functional food using an anchovy processed product according to claim 8, wherein the salted anchovy sauce is prepared by comprising: a filtrate obtaining step of filtering a fermentation product which is produced through the raw material preparation step of preparing anchovy raw material, and the fermentation step of fermenting the prepared raw material; and removing a residue to obtain a filtrate,
wherein the fermentation step is performed by stirring the effective ingredients at 50 rpm to 300 rpm under an anaerobic condition,
the filtrate contains tryptophan among amino acids in an amount of exceeding 5% by weight based on a total amount of the amino acids, and
an oxygen partial pressure in the anaerobic condition is equal to or less than that in the atmosphere.

17. A method of manufacturing a salted fish sauce with increased tryptophan comprising:
a fish and shellfish raw material preparation step of preparing a fish and shellfish raw material;
a fermentation step of fermenting the prepared fish and shellfish raw material; and
a filtrate obtaining step of filtering a fermented product produced through the fermentation step, and removing a residue to obtain a filtrate,
wherein the fermentation step is performed by stirring the raw material at 50 rpm to 300 rpm under an anaerobic condition,
the filtrate contains tryptophan among amino acids in an amount of exceeding 5% by weight based on a total amount of the amino acids, and
an oxygen partial pressure in the anaerobic condition is equal to or less than that in the atmosphere.
